# FASCICULE DE BREVET EUROPEEN

(11) **EP 2 477 965 B1**
(45) Date de publication et mention de la délivrance du brevet: **08.05.2013**
(21) Numéro de dépôt: 10763815.7
(22) Date de dépôt: 06.09.2010
(51) Int. Cl.: C07D 209/42, A61K 31/404, A61P 17/00

(54) **COMPOSITION COSMETIQUE COMPRENANT UN DERIVE DE LEUCODOPACHROME**
KOSMETISCHE ZUSAMMENSETZUNG MIT EINEM LEUKODOPACHROMDERIVAT
COSMETIC COMPOSITION INCLUDING LEUCODOPACHROME DERIVATIVE

(30) Priorité: 16.09.2009 FR 0956365
(43) Date de publication de la demande: 25.07.2012
(73) Titulaire: Chanel Parfums Beauté, 92200 Neuilly-sur-Seine (FR)
(72) Inventeur: DESMURS, Jean-Roger, F-06400 Cannes (FR); DELAIRE, Sabine, F-92500 Rueil Malmaison (FR)
(74) Mandataire: Cabinet Plasseraud
(86) Numéro de dépôt international: PCT/FR2010/051845
(87) Numéro de publication internationale: WO 2011/033207

(56) Documents cités:
- EP-A2- 0 820 767
- DE-A1- 2 153 802

## Description

La présente invention a pour objet une composition cosmétique comprenant un dérivé de leucodopachrome, ainsi que son utilisation comme agent de dépigmentation de la peau. L'invention a également pour objet un procédé de dépigmentation de la peau utilisant ladite composition.

La couleur de la peau humaine est fonction de différents facteurs et notamment des saisons de l'année, de la race et du sexe. Elle est principalement déterminée par la nature et la concentration de mélanine produite par les mélanocytes. Les mélanocytes sont les cellules spécialisées qui, par l'intermédiaire d'organelles particuliers, les mélanosomes, synthétisent la mélanine. En outre, à différentes périodes de leur vie, certaines personnes voient apparaître sur la peau et plus spécialement sur les mains, des taches plus foncées et/ou plus colorées, conférant à la peau une hétérogénéité. Ces taches sont dues aussi à une concentration importante de mélanine dans les kératinocytes situés à la surface de la peau.

L'utilisation de substances dépigmentantes topiques inoffensives présentant une bonne efficacité est tout particulièrement recherchée en vue de traiter les hyperpigmentations régionales par hyperactivité mélanocytaire telles que les mélasmas idiopathiques, survenant lors de la grossesse ("masque de grossesse" ou chloasma) ou d'une contraception oestroprogestative, les hyperpigmentations localisées par hyperactivité et prolifération mélanocytaire bénigne, telles que les taches pigmentaires séniles dites lentigo actiniques, les hyperpigmentations accidentelles, éventuellement dues à la photosensibilisation ou à la cicatrisation post-lésionnelle, ainsi que certaines leucodermies, telles que le vitiligo. Pour ces dernières (les cicatrisations pouvant aboutir a une cicatrice donnant a la peau un aspect plus blanc), à défaut de pouvoir repigmenter la peau lésée, on achève de dépigmenter les zones de peau normale résiduelle pour donner à l'ensemble de la peau une teinte blanche homogène.

Le mécanisme de formation de la pigmentation de la peau, c'est-a-dire de la formation de la mélanine, est particulièrement complexe et fait intervenir schématiquement deux chemins possibles pour l'eumélanine :

### Dopaquinone → leucodopachrome → dopachrome → DHI (5,6-dihydroxyindole ou DHI ) → indole 5,6 quinone

### Dopaquinone → leucodopachrome → dopachrome →DHICA (acide 5,6-dihydroxyindole-2-carboxylique)

La tyrosinase (monophénol oxygénase EC 1.14.18.1: oxygen oxydo-reductase EC 1.14.18.1) est l'enzyme essentielle intervenant dans cette suite de réactions. Elle catalyse notamment la réaction de transformation de la tyrosine en Dopa (dihydroxyphénylalanine) grâce a son activité hydroxylase et la réaction de transformation de la Dopa en dopaquinone grâce à son activité oxydase. Cette tyrosinase n'agit que lorsqu'elle est à l'état de maturation sous l'action de certains facteurs biologiques.

Une substance est reconnue comme dépigmentante ou anti-pigmentante si elle agit directement sur la vitalité des mélanocytes épidermiques où se déroule la mélanogénèse, et/ou si elle interfère avec une des étapes de la biosynthèse de la mélanine soit en inhibant une des enzymes impliquées dans la mélanogénèse, soit en s'intercalant comme analogue structural d'un des composés chimiques de la chaîne de synthèse de la mélanine, chaîne qui peut alors être bloquée et ainsi assurer la dépigmentation.

Les substances cosmétiques les plus utilisées en tant que dépigmentants sont plus particulièrement l'acide ascorbique et ses dérivés, dont le glucoside d'ascorbyle, ainsi que certains extraits de plantes (notamment de réglisse).

Il subsiste néanmoins le besoin d'un nouvel agent blanchissant de la peau humaine ayant une bonne efficacité et qui soit bien toléré.

A cet égard la demanderesse a de manière surprenante et inattendue découvert que certains dérivés de leucodopachrome présentaient une bonne activité dépigmentante. Les dérivés de leucodopachrome sont des composés connus intervenant notamment dans la synthèse de la mélanine. De manière inattendue, les inventeurs ont montré que certains dérivés de leucodopachrome, bien qu'étant des précurseurs de la mélanine, présentaient des propriétés blanchissantes.

De façon plus précise, l'invention a donc pour objet, selon un premier aspect, une composition cosmétique ou dermatologique, caractérisée par le fait qu'elle contient, dans un milieu physiologiquement acceptable, au moins un composé de formule (I) 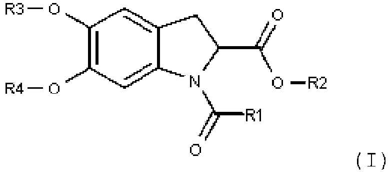 où R1 est
- un hydrogène, ou
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy et /ou acyloxy ;
R2 est :
- un hydrogène, ou
- un alkyle en C1-C18, linéaire ou ramifié, saturé ou insaturé, ou
- un aryle éventuellement substitué, notamment par des groupements acyloxy, ou
- une chaîne polyéthylène glycol (PEG) ou une chaîne polyéthylène glycol monoalkyl éther ;
R3 et R4 sont, indépendamment l'un de l'autre :
- un hydrogène, ou
- un groupement acyle de formule R5-CO- où R5 est un alkyle en C1-C18, linéaire ou ramifié, saturé ou insaturé éventuellement substitué par un ou plusieurs groupements aryle éventuellement substitués, notamment par des groupements acyloxy, ou
- un aryle éventuellement substitué, notamment par des groupements acyloxy.

Les composés de formule (I) incluent les énantiomères de ces composés et leurs mélanges en toutes proportions. L'énantiomère (S) est préféré pour une utilisation dans la présente invention.

L'invention a également pour objet, selon un second aspect, un procédé pour réduire ou prévenir la pigmentation et/ou blanchir et/ou éclaircir la peau, comprenant l'application sur la peau d'une composition cosmétique ou dermatologique, caractérisée par le fait qu'elle contient, dans un milieu physiologiquement acceptable, au moins un composé de formule (I) tel que décrit précédemment.

Selon un troisième aspect, la présente invention a pour objet l'utilisation d'un composé de formule (I) tel que décrit précédemment comme agent dépigmentant et/ou anti-pigmentant et/ou blanchissant et/ou éclaircissant.

Selon un quatrième aspect, l'invention a pour objet un composé de formule (I') 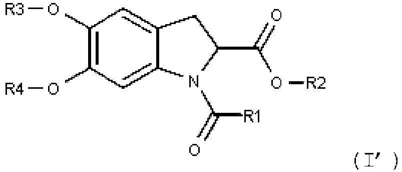 où R1 est
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy et/ou acyloxy ;
R2 est :
- un alkyle en C3-C18, linéaire ou ramifié, saturé ou insaturé, ou
- un aryle éventuellement substitué, notamment par des groupements acyloxy, ou
- une chaîne polyéthylène glycol (PEG) ou une chaîne polyéthylène glycol monoalkyl éther ;
R3 et R4 sont, indépendamment l'un de l'autre :
- un groupement acyle de formule R5-CO- où R5 est un alkyle en C1-C18, linéaire ou ramifié, saturé ou insaturé, éventuellement substitués par un ou plusieurs groupements aryle éventuellement substitués, notamment par des groupements acyloxy, ou
- un aryle éventuellement substitué, notamment par des groupements acyloxy.

### Composés de formule (I) et (I')

Les composés de formule (I) et (I') selon l'invention permettent de dépigmenter et/ou d'éclaircir efficacement la peau d'êtres humains. Ils sont notamment destinés à être appliqués sur la peau d'individus présentant des tâches de pigmentation brunâtres, des taches de sénescence, ou sur la peau d'individus désirant combattre l'apparition d'une couleur brunâtre provenant de la mélanogénèse, par exemple à la suite d'une exposition aux rayonnements ultra-violets.

Pour les composés de formule (I) :
R1 désigne :
   - un hydrogène, ou
   - un alkyle en C1-C18, linéaire ou ramifié, saturé ou insaturé, éventuellement substitué par un ou plusieurs groupements aryle, ou
   - un aryle éventuellement substitué par un ou plusieurs groupes hydroxy et/ou acyloxy ;
R2 désigne :
   - un hydrogène,
   - un alkyle en C1-C18, linéaire ou ramifié, saturé ou insaturé, ou
   - un aryle éventuellement substitué, notamment par des groupements acyloxy, ou
   - une chaîne polyéthylène glycol (PEG) ou une chaîne polyéthylène glycol monoalkyl éther ;
R3 et R4 sont, indépendamment l'un de l'autre :
   - un hydrogène, ou
   - un groupement acyle de formule R5-CO- où R5 est un alkyle en C1-C18, linéaire ou ramifié, saturé ou insaturé, éventuellement substitué par un ou plusieurs groupements aryle éventuellement substitués, notamment par des groupements acyloxy, ou
   - un aryle éventuellement substitué, notamment par des groupements acyloxy.

Selon un mode particulier de réalisation, le composé (I) se présente sous la forme d'un dimère, dont les monomères peuvent être liés de manière covalente via une liaison simple ou par l'intermédiaire d'au moins un atome tel qu'un hétéroatome au radical R1 ou R2 :

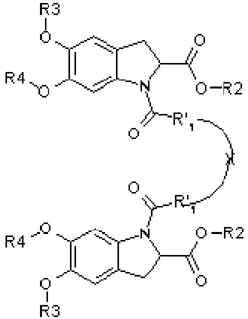

A titre d'exemples non limitatifs du composé (I) selon l'invention, on peut notamment citer :
- Le diméthyl [S-(R*,R*)]-1,1'-[thiobis(1-oxo-3,1-propanediyl)]bis[5,6-bis(acétyloxy)-2,3-dihydro-1H-indole-2-carboxylate de formule 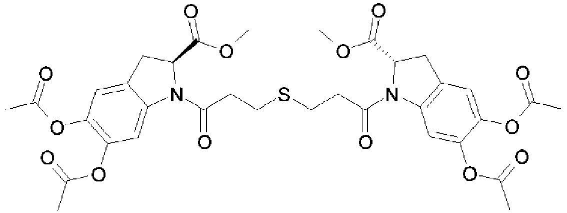
- Le 2,2-diméthyl-1-oxopropoxy)méthyl (S)-5,6-bis(acétyloxy)-1-[3-(acétylthio)-1-oxopropyl]-2,3-dihydro-1H-indole-2-carboxylate de formule 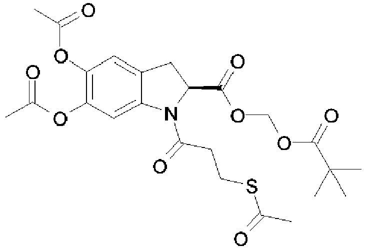
- Le méthyl [S-(R*,R*)]-5,6-bis(acétyloxy)-1-[3-(acétylthio)-2-méthyl-1-oxopropyl]-2,3-dihydro-1H-indole-2-carboxylate de formule 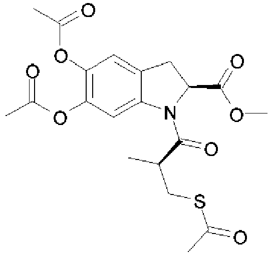
- Le méthyl (S)-5,6-bis(acétyloxy)-2,3-dihydro-1-[1-oxo-3-[(phénylméthyl)thio]propyl]-1H-indole-2-carboxylate de formule 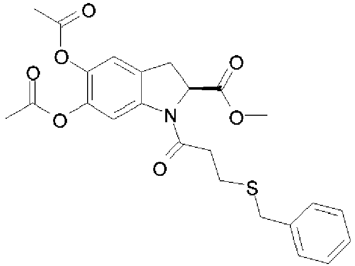
- Le méthyl (S)-5,6-bis(acétyloxy)-1-[3-(benzoylthio)-1-oxopropyl]-2,3-dihydro-1H-indole-2-carboxylate de formule 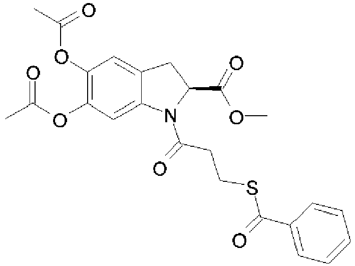 L'ester éthylique de la triacétyl leucodopachrome de formule 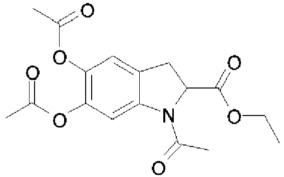
- L'ester méthylique de la triacétyl leucodopachrome de formule

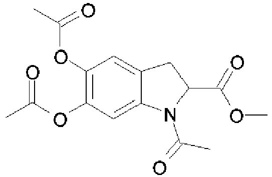

De préférence, pour les composés de formule (I) et (I'), R1 désigne :
- un alkyle en C1-C18, linéaire ou ramifié, saturé ou insaturé éventuellement substitué par un ou plusieurs groupements aryle, ou
- un aryle éventuellement substitué par un ou plusieurs groupes hydroxy et/ou acyloxy.

Préférentiellement, R1 désigne un alkyle en C1-C18 linéaire, et plus préférentiellement encore, R1 est CH3.

Pour les composés de formule (I) et (I'), de préférence, R2 désigne:
- un aryle éventuellement substitué, notamment par des groupements acyloxy,
- une chaîne polyéthylène glycol (PEG) ou une chaîne polyéthylène glycol monoalkyl éther.

Plus préférentiellement, R2 désigne une chaîne polyéthylène glycol (PEG). En effet, la demanderesse a observé que lorsque R2 est une chaîne polyéthylène glycol (PEG), la solubilité du composé (I) ou (I') dans l'eau est améliorée. Il en résulte que le composé (I) ou (I') peut alors être introduit plus facilement dans des compositions cosmétiques comprenant une phase aqueuse.

En variante, dans la formule (I), R2 désigne un alkyle en C1-C18 linéaire, et plus préférentiellement, R2 est -CH3 ou -CH2-CH3.

En outre, dans la formule (I'), R2 peut avantageusement désigner un groupe alkyle linéaire en C3-C18 et plus préférentiellement un groupe n-propyle.

Selon un mode particulièrement préféré de réalisation, pour les composés de formule (I) selon l'invention, R1 désigne -CH3, et R2 désigne -CH3 ou -CH2-CH3.

Préférentiellement, dans la formule (I) ou (I'), R3 et R4 sont, indépendamment l'un de l'autre :
- un groupement acyle de formule R5-CO- où R5 est un alkyle en C1-C18, linéaire ou ramifié, saturé ou insaturé, éventuellement substitués par un ou plusieurs groupements aryle éventuellement substitué, notamment par des groupements acyloxy, ou
- un aryle éventuellement substitué, notamment par des groupements acyloxy.

Selon un mode particulièrement préféré de réalisation, pour les composés de formule (I) selon l'invention, R3 et R4 sont tous les deux des groupements acétyle CH3-CO-.

Selon un mode préféré de réalisation, le composé de formule (I) selon l'invention est l'ester méthylique de triacétyl leucodopachrome (II) ou l'ester éthylique de triacétyl leucodopachrome (III) :

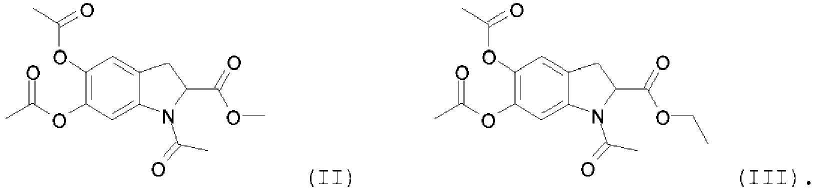

En particulier, la composition selon l'invention comprend l'un des énantiomères ou un mélange racémique (50/50) de deux composés de formule (I) optiquement actifs. Plus préférentiellement, le composé de formule (I) selon l'invention est l'ester méthylique de la (S) triacétyl leucodopachrome ou l'ester éthylique de la (S) triacétyl leucodopachrome.

En outre, un exemple préféré de composé de formule (I') est l'ester propylique de triacétyl leucodopachrome de formule (IV) : 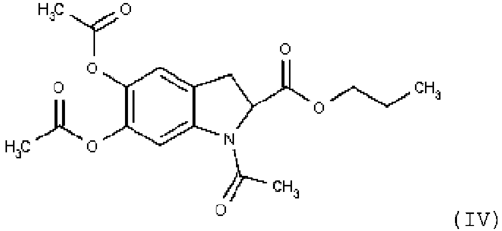 qui peut notamment être présent sous la forme de l'un de ses énantiomères ou d'un mélange racémique (50/50) de deux composés de formule (IV) optiquement actifs.

Le composé de formule (I) peut notamment être présent dans la composition selon l'invention en une teneur allant de 0,00001 à 10% en poids, par rapport au poids total de la composition, et de préférence de 0,001 à 5% en poids.

Les composés de formule (I) peuvent être obtenus par des procédés décrits dans l'art antérieur. On peut citer, à titre d'exemple, les publications de Wyler H., Chiovini J., Helvetica Chimica Acta, 1968, 51, 1476-1494 ; de Woelcke Uwe; Kaiser Ado; Koch Wolfgang; Scheer Marcel, Helvetica Chimica Acta, 1970, 53(7), 1704-1708 ; de Wyler H.; Dreiding Andre S., Helvetica Chimica Acta, 1962, 45, 638-640 ; de Mabry T. J.; Wyler H.; Sassu C.; Mercier M.; Parikh I.; Dreiding Andre S., Helvetica Chimica Acta, 1962, 45, 640-647 ; de Berestovitskaya V. M.; Perekalin V. V.; Sopova A. S., Zhurnal Obshchei Khimiï, 1966, 2(6), 1123-1124 ; de Omote Yoshimori; Fujinuma Yoshimori; Kuo Kung-Tu; Sugiyama Noboru, Nippon Kagaku Zasshi, 1966, 87(7), 760-762 ou de Davies Roger; Laird William M.; Synge Richard L. M., Phytochemistry, 1975, 14(7), 1591-1596.

A titre illustratif et non limitatif, le composé (II) peut être obtenu à partir de la 3,4-dihydroxy-(L)-phénylalanine ci-après appelée (L)-Dopa (V).

Le produit S-(L)-Dopaméthyl ester ou ester méthylique de (L)-Dopa, appelé produit (VI) est obtenu par estérification du produit (L)-Dopa (V), puis subit une oxydation, puis une réduction pour obtenir, après traitement acide, l'ester méthylique de l'acide (S)-5,6-dihydoxy-indoline-2-carboxylique ((L)-Cyclodopa méthyl ester ou (L)-leucodopachrome méthyl ester, produit (VII)).

Alternativement, et toujours de manière non limitative, le composé de formule (II) peut être obtenu à partir de la Bétanine, molécule naturelle extraite de la betterave rouge, selon le procédé de synthèse suivait :

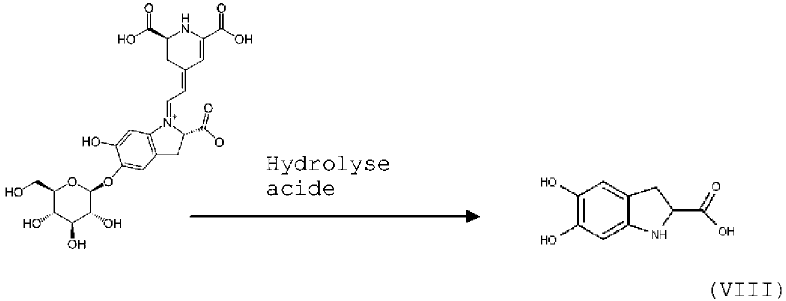

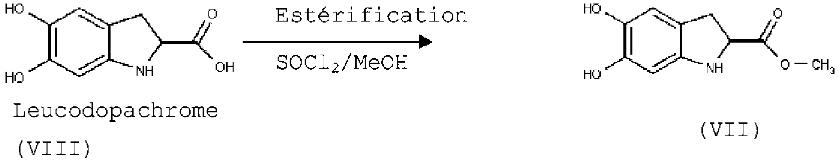

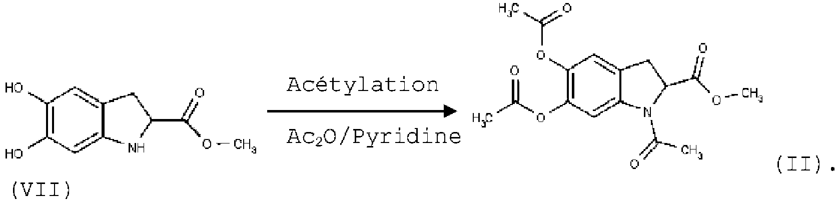

Les étapes d'estérification et d'acétylation permettant de synthétiser l'ester méthylique de la triacétyl leucodopachrome (composé de formule (II)) à partir de la leucodopachrome (VIII) peuvent être analogues à celles du procédé précédemment décrit.

Outre le composé de formule (I) précédemment décrit, la composition selon l'invention peut également comprendre au moins un additif usuel dans le domaine cosmétique, tel que par exemple au moins un composé choisi parmi un agent émollient ou humectant, un agent gélifiant et/ou épaississant, un agent tensioactif, une huile, une cire, un élastomère de silicone, un filtre solaire, un colorant, une charge organique ou inorganique, un conservateur, un agent antioxydant, un agent actif, un séquestrant et un parfum.

Notamment, la composition selon l'invention peut contenir, de manière non limitative, un ou plusieurs des additifs suivantes :
- Un ou plusieurs agent(s) gélifiants(s) et/ou épaississant(s) de la phase aqueuse, choisi par exemple parmi les homo- et copolymères réticulés ou non, hydrophiles ou amphiphiles, d'acide acryloylméthylpropane sulfonique (AMPS) et/ou d'acrylamide et/ou d'acide acrylique et/ou de sels ou d'esters d'acide acrylique tels que les ammonium acryloyldiméthyltaurate/vp copolymer et ammonium acryloyldiméthyl-taurate/beheneth-25 méthacrylate copolymer, notamment ceux vendus sous les dénominations Aristoflex AVC et HMB de Clariant, les dérivés cellulosiques, les gommes d' origine végétale (guar, caroube, alginates, carraghénanes, pectine) ou d'origine microbienne (xanthane), les argiles (laponite).
   Ledit agent gélifiant et/ou épaississant peut être présent dans la composition en une teneur de l'ordre de 0,01 à 5% en poids, par rapport au poids total de la composition ;
- Un ou plusieurs agent(s) tensioactif(s), de préférence émulsionnants, qu'ils soient non ioniques, anioniques, cationiques ou amphotères, et en particulier les esters d'acides gras et de polyols tel que les esters d'acides gras et de glycérol, les esters d'acides gras et de sorbitan, les esters d'acides gras et de polyéthylèneglycol et les esters d'acides gras et de sucrose; les éthers d'alcools gras et de polyéthylèneglycol; les alkylpolyglucosides; les polysiloxanes modifiés polyéthers; la bétaïne et ses dérivés; les polyquaterniums; les sels de sulfate d'alcools gras éthoxylés; les sulfosuccinates; les sarcosinates; les alkyl- et dialkylphosphates et leurs sels; et les savons d'acides gras. Ledit agent tensioactif peut être présent dans la composition dans une teneur de l'ordre de 0,1 à 8%, de préférence 0,5 à 3% en poids, par rapport au poids total de la composition ;
- Un ou plusieurs co-tensioactif(s) tels que les alcools gras linéaires et en particulier les alcools cétylique et stéarylique;
- Un ou plusieurs corps gras liquide(s) à température ambiante, communément dénommé(s) huiles(s), volatile(s) ou non volatile(s), hydrocarboné(s), siliconée(s), linéaire(s), cyclique(s) ou ramifiée(s), par exemple, les huiles de silicone telles que les polydiméthylsiloxanes (diméthicones), les polyalkylcyclosiloxanes (cyclométhicones) et les polyalkylphénylsiloxanes (phenyldiméthicones) ; les huiles synthétiques telles que les huiles fluorées, les alkyl benzoates et les hydrocarbures ramifiés tels que le polyisobutylène; les huiles végétales et notamment de soja ou de jojoba; et les huiles minérales telles que l'huile de paraffine ; de préférence à raison de 0,1 à environ 10%, mieux, de 0,5 à 5% en poids, par rapport au poids total de la composition ;
- Une ou plusieurs cires, telles que l'ozokérite, la cire de polyéthylène, la cire d'abeille ou la cire de carnauba, de préférence à raison de 0,01 à environ 5%, de préférence 0,5 à 5% en poids, par rapport au poids total de la composition ;
- Un ou plusieurs élastomère(s) de silicone obtenus notamment par réaction, en présence d'un catalyseur, d'un polysiloxane ayant au moins un groupe réactif (hydrogène ou vinyle, notamment) et portant au moins un groupe alkyle (notamment méthyle) ou phényle, terminal et/ou latéral, avec un organosilicone tel qu'un organohydrogénopolysiloxane, de préférence à raison de 0,1 à environ 20%, de préférence 0,25 à 15% en poids, par rapport au poids total de la composition ;
- Un ou plusieurs filtre(s) solaire(s), notamment les filtres organiques, tels que les dérivés de dibenzoylméthane (dont le butyl méthoxydibenzoylméthane vendu en particulier par DSM sous le nom commercial Parsol 1789), les dérivés d'acide cinnamique (dont l'éthylhexyl méthoxycinnamate vendu en particulier par DSM sous le nom commercial Parsol MCX), les salicylates, les acides para-aminobenzoïques, les β-β'-diphénylacrylates, les benzophénones, les dérivés de benzylidène camphre, les phénylbenzimidazoles, les triazines, les phénylbenzotriazoles et les dérivés anthraniliques; ou les filtres inorganiques, à base d'oxydes minéraux sous forme de pigments ou de nanopigments, enrobés ou non, et en particulier à base de dioxyde de titane ou d'oxyde de zinc; de préférence à raison de 0,1 à environ 30%, mieux, de 0,5 à 20% en poids, par rapport au poids total de la composition ;
- Un ou plusieurs colorant(s) hydrosoluble(s) tels que, par exemple, le sel disodique de ponceau, le sel disodique du vert d' alizarine, le jaune de quinoléine, le sel trisodique d'amarante, le sel disodique de tartrazine, le sel monosodique de rhodamine, le sel disodique de fuchsine ou la xanthophylle, de préférence à raison de 0,1 à environ 2% en poids, par rapport au poids total de la composition,
- Une ou plusieurs charge(s), et en particulier les poudres à effet soft-focus, qui peuvent être notamment choisies parmi les polyamides, la silice, le talc, le mica, les fibres (notamment de polyamide ou de cellulose);
- Un ou plusieurs conservateur(s) ;
- les séquestrants tels que les sels d'EDTA;
- les parfums;
- et leurs mélanges.

Des exemples de tels adjuvants sont cités notamment dans le Dictionnaire CTFA (International Cosmetic Ingredient Dictionary and Handbook publié par The Cosmetic, Toiletry and Fragrance Association, 11ème Edition, 2006) qui décrit une grande variété, sans limitation, d'ingrédients cosmétiques et pharmaceutiques habituellement utilisés dans l'industrie du soin de la peau, qui conviennent pour être utilisés comme ingrédients additionnels dans les compositions selon la présente invention.

L' homme du métier est en mesure de choisir, parmi l'ensemble de ces éventuels additifs, aussi bien la composition que la quantité de ceux qui seront ajoutés à la composition, de telle sorte que celle-ci conserve l'ensemble de ses propriétés.

En outre, la composition selon la présente invention peut éventuellement contenir divers agents actifs qui peuvent être choisis dans le groupe constitué des vitamines, des antioxydants, des agents hydratants, des émollients, des agents anti-vieillissement, des agents anti-pollution, les agents kératolytiques, des astringents, des anti-inflammatoires non stéroïdiens et des agents blanchissants.

Des exemples de vitamine incluent les vitamines A, B1, B2, B6, C et E et leurs dérivés, l'acide pantothénique et ses dérivés et la biotine.

Des exemples d'antioxydants incluent l'acide ascorbique et ses dérivés tels que le palmitate d'ascorbyle, le tétraisopalmitate d' ascorbyle, l'ascorbyl glucoside, le magnésium ascorbyl phosphate, le sodium ascorbyl phosphate et le sorbate d' ascorbyle; le tocophérol et ses dérivés, tels que l'acétate de tocophérol, le sorbate de tocophérol et d'autres esters de tocophérol; le BHT et BHA; le dibutyle hydroxytoluène, le butyl hydroxyanisole, les esters de l'acide gallique, l'acide phosphorique, l'acide citrique, l'acide maléique, l'acide malonique, l'acide succinique, l'acide fumarique, la céphaline, l'hexamétaphosphate, l'acide phytique, l'acide tétraacétique d'éthylènediamine et les extraits de plantes, par exemple de Chondrus cripsus, Rhodiola, Thermus thermophilus, la feuille de maté, le bois de chêne, l'écorce de Rapet Kayu, les feuilles de Sakura et les feuilles d'ylang ylang.

Des exemples d'agents hydratants incluent le polyéthylène glycol, le propylène glycol, le dipropylène glycol, la glycérine, le 1,3-butylène glycol, le xylitol, le sorbitol, le maltitol, les mucopolysaccharides, tels que l'acide chondroïtine sulfurique, l'acide hyaluronique, et l'acide mucoitinsulfurique; l'acide caronique; l'atelo collagène; le chlorestéryl-12-hydroxystéarate; les sels biliaires, une composante principale du FHN (facteur d'hydratation naturelle) comme un sel de l'acide pyrrolidone carboxylique et un sel d'acide lactique, un analogue d'acide aminé tel que l'urée, la cystéine et la sérine; un collagène soluble à chaîne courte, les PPG diglycérine, les homo- et copolymères de 2-méthacryloyloxyéthylphosphorylcholine comme le Lipidure HM et le Lipidure PBM de NOF; l'allantoïne; le P E G / P P G / polybutylène Glycol-8/5/3 Glycérine de NOF vendu sous la dénomination commerciale Wilbride S 753; la triméthylglycine vendu sous la dénomination commerciale Aminocoat par la société Ashahi KASEI Chemicals et divers extraits de plantes tels que des extraits de Castanea sativa, des protéines de noisette hydrolysées, les polysaccharides tuberosa Polyanthes, l'huile de noyau d'Argania spinosa et les extraits de nacre contenant un conchyoline qui est vendu notamment par la compagnie Maruzen (Japon) sous le nom commercial Pearl Extract®.

Des exemples d'émollients incluent le polyméthacrylate de glycéryle; le gluceth méthyl-20.

Des exemples d'agents anti-vieillissement incluent les acides aminés acylés (par exemple le Maxilip, le Matrixyl 3000 ou le biopeptide CL de SEDERMA ou le Sepilift de SEPPIC), des extraits de Pisum sativum, des protéines de soja hydrolysées, des dérivés de méthylsilanol tels que le mannuronate de méthylsilanol, des tourteaux hydrolysés de Cucurbita pepo, des extraits de Scenedesmus.

Des exemples d'agents anti-pollution incluent l'extrait de graines de Moringa pterygosperma (par exemple Purisoft de LSN); l'extrait de beurre de karité (par exemple Detoxyl de SILAB), un mélange d'extrait de lierre, l'acide phytique, l'extrait de graine de tournesol (par exemple OSMOPUR de SEDERMA).

Des exemples d'agents kératolytiques incluent les α-hydroxyacides (par exemple les acides glycolique, lactique, citrique, malique, mandélique, ou tartrique) et les β-hydroxyacides (par exemple l'acide salicylique), et leurs esters, tels que les C12-13 alkyl lactates, et les extraits de plantes contenant ces hydroxyacides, tels que des extraits d'Hibiscus sabdriffa.

Des exemples d'astringents incluent les extraits d'hamamélis.

Des exemples d'agents anti-inflammatoires incluent le bisabolol, l'allantoïne, l'acide tranexamique, l'oxyde de zinc, l'oxyde de soufre et ses dérivés, le sulfate de chondroïtine, l'acide glycyrrhizinique et ses dérivés tels que les glycyrrhizinates.

Outre le dérivé de leucodopachrome, la composition selon l'invention peut comprendre un agent blanchissant additionnel capable de bloquer la synthèse des protéines structurelles intervenant dans le mécanisme de la mélanogénèse (phase I) tels que les glycoprotéines spécifiques des mélanocytes Pme117. Un tel agent actif peut être l'acide férulique, le Cytovector® (eau, glycol, lécithine, acide férulique, hydroxyéthylcellulose) commercialisé par BASF.

Les compositions cosmétiques selon la présente invention peuvent également contenir au moins un peptide tel que décrit dans la demande de brevet WO2009010356, un bioprécurseur tel que décrit dans la demande de brevet WO2006134282 ou un sel de tranexamate tel que le sel de chlorhydrate de tranexamate cétylique.

Outre le dérivé de leucodopachrome, la composition selon l'invention peut comprendre un agent blanchissant additionnel ayant un effet d'inhibition de la synthèse de mélanine et/ou un effet d'inhibition de l'expression MITF et/ou une activité anti-tyrosinase et/ou un effet d'inhibition de la synthèse d'endothéline-1, tel qu'un extrait de réglisse (extrait de Glycyrrhiza glabra), qui est vendu notamment par la société Maruzen sous le nom commercial Licorice extract®.

En variante ou en plus, la composition selon l'invention peut comprendre un agent blanchissant additionnel ayant également un effet antioxydant, comme les composés de vitamine C, y compris les sels d'ascorbate, les esters ascorbyle d'acides gras ou d'acide sorbique, et d'autres dérivés de l'acide ascorbique, par exemple, les phosphates d'ascorbyle, tels que le magnésium ascorbyl phosphate et sodium ascorbyl phosphate, ou les esters de saccharide d'acide ascorbique, qui incluent, par exemple, l'ascorbyle-2-glucoside, le L-ascorbate de 2-O-alpha-D-glucopyranosyl, ou le L-ascorbate de 6-O-bêta-D-galactopyranosyl. Un agent actif de ce type est vendu en particulier par la société DKSH sous le nom commercial Ascorbyl glucoside ®.

D'autres agents de blanchiment peuvent encore être inclus dans les compositions selon la présente invention. On peut citer des agents dépigmentants tels que les extraits de plantes, y compris des extraits de Narcissus tazetta; d'arbutine, d'acide kojique, d'acide ellagique; de cystéine; de 4-thioresorcin; de résorcinol ou de rucinol ou leurs dérivés, de l'acide glycyrrhizinique et d'hydroquinone-bêta-glucoside.

Avantageusement, la composition cosmétique selon l'invention peut se présenter sous la forme d'une poudre, d'une émulsion, d'une microémulsion, d'une nanoémulsion, d'une suspension, d'une solution d'une lotion, d'une crème, d'un gel aqueux ou hydroalcoolique, d'une mousse, d'un sérum, d'une solution ou d'une dispersion pour aérosol, ou d'une dispersion de vésicules lipidiques.

Dans le cas d'une émulsion, il peut s'agir d'une émulsion eau-dans-huile ou huile-dans-eau.

Dans le procédé selon l'invention, la composition peut être appliquée le matin et/ou le soir sur la peau du visage, du décolleté et/ou des mains, et de préférence sur les zones hyperpigmentées.

L'invention est illustrée de manière non limitative par les exemples ci-dessous.

### EXEMPLES

### Exemple 1 : Synthèse d'un dérivé de leucodopachrome : l'ester méthylique de la (S)-triacétyl-leucodopachrome (II)

Le composé (II) est obtenu à partir de la 3,4-dihydroxy-(L)-phénylalanine ci-après appelée (L)-Dopa (V).

Le produit S-(L)-Dopaméthyl ester ou ester méthylique de (L)-Dopa, appelé produit (VI) est obtenu par estérification du produit (L)-Dopa (V), puis subit une oxydation, puis une réduction pour obtenir, après hydrolyse acide, l'ester méthylique de l'acide (S)-5,6-dihydoxy-indoline-2-carboxylique ((L)-Cyclodopa méthyl ester ou (L)-leucodopachrome méthyl ester, produit (VII)).

En particulier, la S-(L)-Dopa- méthyl ester (VI) est obtenue de la manière suivants :

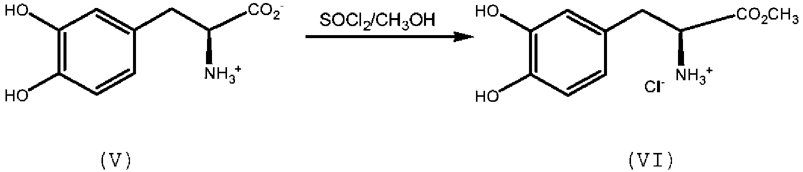

Dans un ballon sous atmosphère d'azote, 20 mL de méthanol anhydre sont refroidis à -5 °C à l'aide d'un bain de glace et de NaCl. Le chlorure de thionyle (5 mL, 14 éq) est ajouté goutte à goutte.

La L-Dopa (V) (1 g, 50 mmol) est ajoutée au milieu réactionnel. Celui ci est agité pendant 1 h à température ambiante puis porté au reflux pendant 1 h. Le solvant est évaporé.

L'ester obtenu est un chlorhydrate et se présente sous la forme d'un solide blanc.

L'ester méthylique de leucodopachrome (VII) peut alors être obtenu selon le schéma réactionnel suivait :

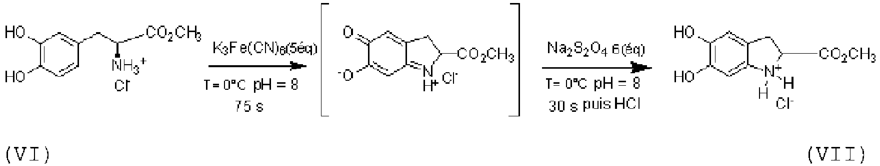

Dans un ballon, le S-(L)-Dopa-méthyl ester (VI) (500 mg, 2,02 mmol, 1 éq) est dissout dans 150 mL d'une solution tampon aqueux phosphate (pH= 8, pour 1 L de solution : 0,5 g de KH2PO4 et 2 2 , 6 g de Na2HPO4.7H2O sont dissouts dans de l'eau distillée).

Le milieu réactionnel est refroidi à l'aide d'un bain de glace à 0°C. Une solution de K3Fe(CN)6 (3,325 g, 10,1 mmol, 5 éq.) dans 100 mL de tampon phosphate est refroidie par le bain de glace puis ajoutée en une fois au milieu réactionnel.

Après 75 s, 3,24 g de Na2S2O4 à 85% en masse (12,4 mmol, 6 éq) dans 50 mL de solution tampon (refroidie par le bain de glace) sont ajoutés.

Après 30 s, 5 mL de HCl à 37% sont additionnés. L'eau est évaporée sous pression réduite. Le résidu solide est trituré deux fois par 10 mL de toluène. La phase toluénique est concentrée par évaporation du toluène.

On obtient un résidu solide de couleur marron pâle contenant l'ester méthylique de **(S)**-leucodopachrome (VII) et les sels du milieu réactionnel.

L'ester méthylique de (S)-triacétyl leucodopachrome (II) est alors obtenu par acétylation du produit (VII) :

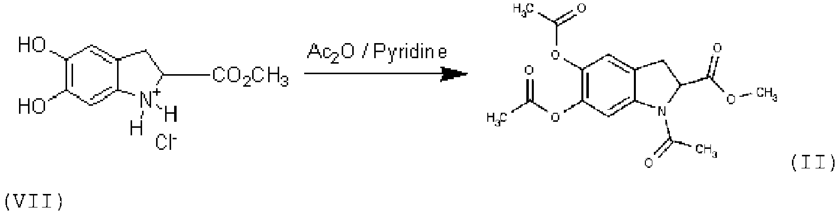

Le résidu de l'étape précédente est placé sous vide puis sous atmosphère d'azote, 25 mL de pyridine et 25 mL d'anhydride acétique sont ajoutés. Le milieu réactionnel est trituré pour casser les agglomérats solides, puis agité pendant 4 h à température ambiante.

Le milieu réactionnel est ensuite filtré sur célite et lavé avec 150 mL de dichlorométhane avant évaporation du solvant.

Du dichlorométhane est ajouté au résidu (50 mL) puis 50 mL d'une solution 1M d'HCl. La phase organique est traitée avec une solution saturée de NaHCO3, par H2O jusqu'à pH neutre, puis par une solution saturée en NaCl. La phase organique est séchée sur MgSO4, le solvant est éliminé par évaporation rotative.

Le produit désiré est obtenu sous forme d'un solide marron avec un rendement de 77% par rapport au S-(L)-Dopa-méthyl ester (2). L'ester méthylique de (S)-triacétyl-leucodopachrome (II) est purifié sur colonne de silice (dichlorométhane/méthanol (19/1)).

### Exemple 2 : Synthèse d'un dérivé de leucodopachrome : l'ester, propylique de (S)-triacétyl-leucodopachrome (XI)

Le composé (XI) est obtenu à partir de la 3,4-dihydroxy-(L)-phénylalanine ci-après appelée (L)-Dopa (V).

Le produit S-(L)-Dopa-propyl ester ou ester propylique de (L)-Dopa, appelé produit (IX) est obtenu par estérification du produit (L)-Dopa (V), puis subit une oxydation, puis une réduction pour obtenir, après hydrolyse acide, l'ester propylique de l'acide (S)-5,6-dihydoxy-indoline-2-carboxylique ((L)-Cyclodopa propyl ester ou (L)-leucodopachrome propyl ester, produit (X)).

En particulier, la S-(L)-Dopa-propyl ester (IX) est obtenue de la manière suivants :

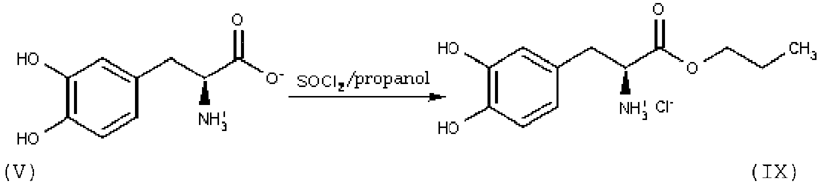

Dans un ballon sous atmosphère d'azote, 20 mL de propanol anhydre sont refroidis à -5 °C à l'aide d'un bain de glace et de NaCl. Le chlorure de thionyle (5 mL, 2 éq) est ajouté goutte à goutte.

La L-Dopa (V) (1 g, 50 mmol) est ajoutée au milieu réactionnel. Celui ci est agité pendant 1 h à température ambiante puis porté au reflux pendant 1 h. Le solvant est évaporé.

L'ester obtenu est un chlorhydrate et se présente sous la forme d' un solide blanc.

L'ester propylique de (S)-leucodopachrome (X) peut alors être obtenu selon le schéma réactionnel suivait :

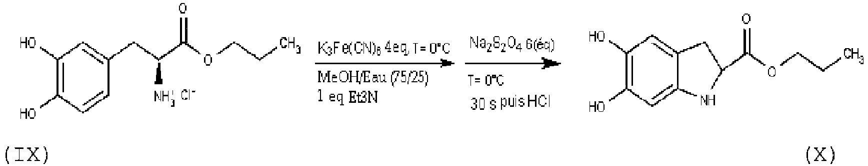

Dans un ballon, le S-(L)-Dopa-propyl ester (IX) (500 mg, 2,02 mmol, 1 éq) est dissout dans 150 mL d'une solution MeOH/H₂O (75/25) avec 1 eq de Et₃N (triéthylamine).

Le milieu réactionnel est refroidi à l'aide d'un bain de glace à 0°C. Une solution de K3Fe(CN)6 (3,325 g, 10,1 mmol, 4 éq.)

Après 75 s, 3,24 g de Na2S204 à 85% en masse (12,4 mmol, 6 éq) dans 50 mL de solution MeOH/H₂O,

Après 10 min, 5 mL de HCl à 3% sont additionnés. L'eau est évaporée sous pression réduite. Le résidu solide est trituré deux fois par 10 mL de toluène. La phase toluénique est concentrée par évaporation du toluène.

On obtient un résidu solide de couleur marron pâle contenant l'ester propylique de (S)-leucodopachrome (X) et les sels du milieu réactionnel.

L'ester propylique de la (S)-triacétyl leucodopachrome (XI) (ou ester méthylique de triacétyl-leucodopachrome) est alors obtenu par acétylation du produit (X) :

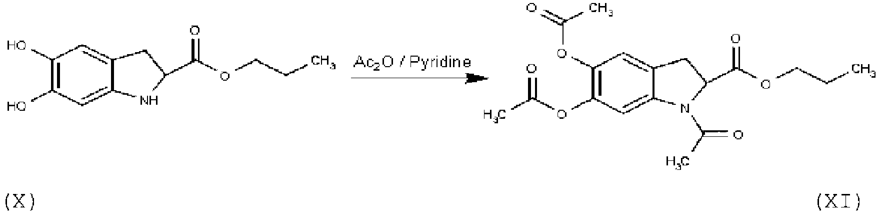

Le résidu de l'étape précédente est placé sous vide puis sous atmosphère d'azote, 25 mL de pyridine et 25 mL d'anhydride acétique sont ajoutés. Le milieu réactionnel est trituré pour casser les agglomérats solides, puis agité pendant 4 h à température ambiante.

Le milieu réactionnel est ensuite filtré sur célite et lavé avec 150 mL de dichlorométhane avant évaporation du solvant.

Du dichlorométhane est ajouté au résidu (50 mL) puis 50 mL d'une solution 1M d'HCl. La phase organique est traitée avec une solution saturée de NaHCO3 par H2O jusqu' à pH neutre, puis par une solution saturée en NaCl. La phase organique est séchée sur MgSO4, le solvant est éliminé par évaporation rotative.

Le produit désiré est obtenu sous forme d'un solide marron avec un rendement de 72% par rapport au S-(L)-Dopa-propyl ester (IX). L'ester propylique de (S)-triacétyl-leucodopachrome (XI) est purifié sur colonne de silice (dichlorométhane/méthanol (19/1)).

### Exemple 3 : Emulsion huile-dans-eau

On prépare une émulsion huile-dans-eau ayant la composition suivants :

| **Composés** | **Dénomination Commerciale** | **Fournisseur** | **TENEUR (en %)** |
|---|---|---|---|
| Eau déminéralisée | | | 72, 14 |
| Mélange d'eau, de sodium phytate et d'alcool | DERMOFEEL PA-3 | LUCAS MEYER | 0,1 |
| Polymère réticulé acrylate/acrylate d'alkyle en C10-C30 | CARBOPOL ULTREZ 21 | GATEFOSSE | 0,2 |
| Mélange d'alcool cétylique, de glycéryl stéarate, de stéarate de PEG 75, de ceteth-20 et de steareth-20 | EMULIUM DELTA | GATEFOSSE | 4 |
| Triheptanoine | DERMOFEEL TC-7 | LUCAS MEYER | 2,5 |
| Squalane | PHYTOSQUALAN | | 3 |
| Tocophéryl acétate | DL-ALPHA-TOCOPHERYL ACETATE | DSM | 0,2 |
| Phénoxyéthanol | PHENOXYETHANOL S | | 0,7 |
| Méthyl triméthicone | TMF-1.5 | DKSH | 6 |
| Butylène glycol | 1,3 BUTYLENE GLYCOL | | 4 |
| Méthylpropanediol | DUB DIOL | STEARINERIE DUBOIS | 2 |
| Alcool | ALCOOL EXPORT A 96°2 | | 5 |
| Composé de formule (I) : ester méthylique de la triacétyl leucodopachrome | | | 0,1 |
| Hydroxyde de sodium | SOLUTION DE SOUDE A 25% | | 0,06 |

Le produit appliqué sur la peau permet un blanchiment et un éclaircissement de celle-ci.

### Exemple 4 : Gel hydroalcoolique

On prépare un gel hydroalcoolique ayant la composition suivants :

| **Composés** | **Dénomination Commerciale** | **Fournisseur** | **TENEUR (en %)** |
|---|---|---|---|
| Eau déminéralisée | | | 83,55 |
| Mélange d'eau, de sodium phytate et d'alcool | DERMOFEEL PA-3 | LUCAS MEYER | 0,1 |
| Mélange de tétraisostéarate de sorbeth-30, sesquiostérarate de sorbitan, PPG-8-Ceteth-20, copolymère acrylate/ méthacrylate de beheneth-20, dipropylène glycol et eau | NIKKOMULESE SE | GATEFOSSE | 4,5 |
| Phénoxyéthanol | PHENOXYETHANOL S | COGNIS CORP | 0,7 |
| Butylène glycol | 1,3 BUTYLENE GLYCOL | | 4 |
| Méthylpropanediol | DUB DIOL | STEARINERIE DUBOIS | 2 |
| Alcool | ALCOOL EXPORT A 96°2 | | 5 |
| Composé de formule (I) : ester méthylique de la triacétyl leucodopachrome | | | 0,1 |
| Hydroxyde de potassium | POTASSE PASTILLE PURE 85-8 | | 0,05 |

Le gel appliqué sur la peau permet un blanchiment et un éclaircissement de celle-ci.

### Exemple 5 : effet de l'ester méthylique de la triacétyl leucodopachrome sur la synthèse de mélanine

On a préparé 3 solutions d'ester méthylique de la triacétyl leucodopachrome, respectivement à des concentrations de 0.0025%, 0.00025% et 0.000025% en poids dans du DMSO.

On a évalué l'effet de l'ester méthylique de la (S)-triacétyl leucodopachrome sur la synthèse de mélanine selon le protocole opératoire suivant.

Des mélanocytes hyperpigmentés dérivés de prépuce néonatal normal de donateurs isolés ont été achetés auprès de la société Cascade Biologics (Portland, Orégon, Etats-Unis). Les cellules ont été ensemencées dans des plaques à 6 puits et cultivées dans un milieu de croissance des mélanocytes (Milieu complété avec de l'extrait pituitaire bovin, sérum foetal bovin, de l'insuline bovine, de la transferrine, facteur de croissance des fibroblasts classique, l'hydrocortisone, l'héparine et phorbol 12-myristate 13-acétate).

Dans un second temps, les cellules ont été incubées avec un milieu de croissance (MGM, Clonetics) contenant les produits actifs, en trois exemplaires, pour 5 jours. Le milieu a été retiré et les cellules lavées avec du PBS (Gibco / Invitrogen). Les cellules ont été lysées et la mélanine solubilisée dans NaOH.

Le surnageant a été centrifugé. Après centrifugation, le lysat clair a été transféré dans une nouvelle plaque et la teneur en mélanine a été mesurée sur un lecteur ELISA à 490 nm. Les culots ont été visualisés sur un imageur AlphaInnotech.

Les résultats sont les suivantes :

| Teneur en ester méthylique de la (S)-triacétyl leucodopachrome | % d' inhibition de la synthèse de mélanine |
|---|---|
| 0.0025% | 32.76 |
| 0.00025% | 6.70 |
| 0.000025% | 5.49 |

On observe une inhibition de la synthèse de mélanine, qui augmente avec la concentration en ester méthylique de la (S)-triacétyl leucodopachrome dans la solution.

## Revendications

1. Composition cosmétique ou dermatologique, **caractérisée par le fait qu'**elle contient, dans un milieu physiologiquement acceptable, au moins un composé de formule (I) 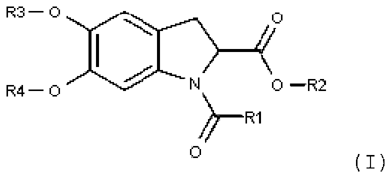 où R1 est
- un hydrogène, ou
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy et/ou acyloxy ;
R2 est :
- un hydrogène, ou
- un alkyle en C1-C18, linéaire ou ramifié, saturé ou insaturé, ou
- un aryle éventuellement substitué, notamment par des groupements acyloxy, ou
- une chaîne polyéthylène glycol (PEG) ou une chaîne polyéthylène glycol monoalkyl éther ;
R3 et R4 sont, indépendamment l'un de l'autre :
- un hydrogène, ou
- un groupement acyle de formule R5-CO- où R5 est un alkyle en C1-C18, linéaire ou ramifié, saturé ou insaturé, éventuellement substitué par un ou plusieurs groupements aryle éventuellement substitués, notamment par des groupements acyloxy, ou
- un aryle éventuellement substitué, notamment par des groupements acyloxy.

2. Composition selon la revendication 1, **caractérisée par le fait que** :
R1 désigne
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy et/ou acyloxy ;
R2 désigne
- un alkyle en C1-C18, linéaire ou ramifié, saturé ou insaturé,
- un aryle éventuellement substitué, notamment par des groupements acyloxy,
- une chaîne polyéthylène glycol (PEG) ou une chaîne polyéthylène glycol monoalkyl éther.

3. Composition selon l'une quelconque des revendications 1 ou 2, **caractérisée par le fait que** R2 est une chaine polyéthylène glycol (PEG).

4. Composition selon l'une quelconque des revendications 1 ou 2, **caractérisée par le fait que** R1 est -CH3 et R2 est -CH3 ou -CH2-CH3.

5. Composition selon l'une quelconque des revendications 1 à 4, **caractérisée par le fait que** R3 et R4 sont, indépendamment l'un de l'autre :
- un groupement acyle de formule R5-CO- où R5 est un alkyle en C1-C18, linéaire ou ramifié, saturé ou insaturé, éventuellement substitué par un ou plusieurs groupements aryle éventuellement substitués, notamment par des groupements acyloxy, ou
- un aryle éventuellement substitué, notamment par des groupements acyloxy.

6. Composition selon l'une quelconque des revendications 1 à 5, **caractérisée par le fait que** R3 et R4 sont tous les deux des groupements acétyle CH3-CO-.

7. Composition selon l'une quelconque des revendications précédentes, **caractérisée par le fait que** le composé de formule (I) est l'ester méthylique de triacétyl leucodopachrome (II) ou l'ester éthylique de triacétyl leucodopachrome (III) :

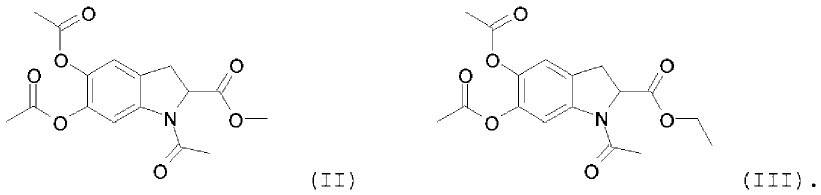

8. Composition selon l'une quelconque des revendications précédentes, **caractérisée par le fait qu'**elle comprend l'un des énantiomères ou un mélange racémique (50/50) de deux composés de formule (I) optiquement actifs.

9. Composition selon l'une quelconque des revendications précédentes, **caractérisée par le fait que** le composé de formule (I) est présent en une teneur allant de 0,00001 à 10% en poids et de préférence de 0,001 à 5% en poids par rapport au poids total de la composition.

10. Procédé pour réduire ou prévenir la pigmentation et/ou blanchir et/ou d'éclaircir la peau, comprenant l'application sur la peau d'une composition cosmétique ou dermatologique, **caractérisée par le fait qu'**elle contient, dans un milieu physiologiquement acceptable, au moins un composé de formule (I). 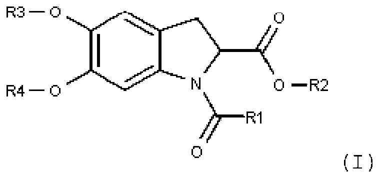 où R1 est
- un hydrogène, ou
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy et/ou acyloxy ;
R2 est :
- un hydrogène, ou
- un alkyle en C1-C18, linéaire ou ramifié, saturé ou insaturé, ou
- un aryle éventuellement substitué, notamment par des groupements acyloxy, ou
- une chaîne polyéthylène glycol (PEG) ou une chaîne polyéthylène glycol monoalkyl éther ;
R3 et R4 sont, indépendamment l'un de l'autre :
- un hydrogène, ou
- un groupement acyle de formule R5-CO- où R5 est un alkyle en C1-C18, linéaire ou ramifié, saturé ou insaturé, éventuellement substitué par un ou plusieurs groupements aryle éventuellement substitués, notamment par des groupements acyloxy, ou
- un aryle éventuellement substitué, notamment par des groupements acyloxy ;
ladite composition étant selon l'une quelconque des revendications 1 à 9.

11. Utilisation d'un composé de formule (I) 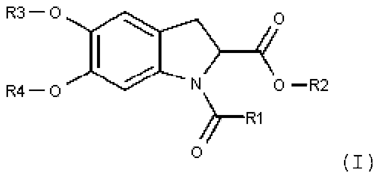 dans laquelle R1, R2, R3, et R4 sont tels que définis dans les revendications 1 à 8, comme agent dépigmentant et/ou anti-pigmentant et/ou blanchissant et/ou éclaircissant.

12. Utilisation selon la revendication 11, pour la fabrication d'une composition destinée à inhiber la synthèse de la mélanine.

13. Composé de formule (I') 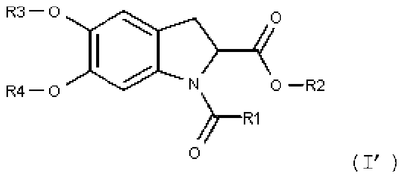 où R1 est
- un alkyle en C1-C18, linéaire ou ramifié, saturé ou insaturé, éventuellement substitué par un ou plusieurs groupements aryle, ou
- un aryle éventuellement substitué par un ou plusieurs groupes hydroxy, acyloxy ;
R2 est :
- un alkyle en C3-C18, linéaire ou ramifié, saturé ou insaturé, ou
- un aryle éventuellement substitué, notamment par des groupements acyloxy, ou
- une chaîne polyéthylène glycol (PEG) ou une chaîne polyéthylène glycol monoalkyl éther ;
R3 et R4 sont, indépendamment l'un de l'autre :
- un groupement acyle de formule R5-CO- où R5 est un alkyle en C1-C18, linéaire ou ramifié, saturé ou insaturé, éventuellement substitué par un ou plusieurs groupements aryle éventuellement substitué, notamment par des groupements acyloxy, ou
- un aryle éventuellement substitués, notamment par des groupements acyloxy.

14. Composé selon la revendication 13, **caractérisé par le fait que** R2 est :
- un groupement alkyl linéaire en C3-C18, ou
- une chaîne polyéthylène glycol (PEG) ou une chaîne polyéthylène glycol monoalkyl éther.

15. Composé selon l'une quelconque des revendications 13 ou 14, **caractérisé par le fait que** le composé de formule (I') est l'ester propylique de triacétyl leucodopachrome de formule (IV) 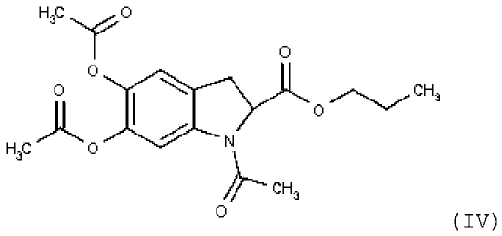 éventuellement sous la forme de l'un des énantiomères ou d'un mélange racémique (50/50) de deux composés de formule (IV) optiquement actifs.

## Patentansprüche

1. Kosmetische oder dermatologische Zusammensetzung,
**dadurch gekennzeichnet, dass** sie in einer physiologisch akzeptablen Umgebung mindestens eine Verbindung der Formel (I) enthält, 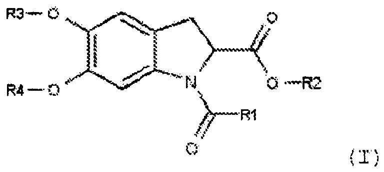 worin
R1 darstellt:
- Wasserstoff, oder
- ein lineares oder verzweigtes, gesättigtes oder ungesättigtes, wahlweise mit einer oder mehreren Arylgruppen substituiertes C1-C18-Alkyl, oder
- ein wahlweise mit einer oder mehreren Hydroxy- und/oder Acyloxygruppen substituiertes Aryl;
R2 darstellt:
- Wasserstoff, oder
- ein lineares oder verzweigtes, gesättigtes oder ungesättigtes C1-C18-Alkyl, oder
- ein wahlweise, insbesondere mit Acyloxygruppen, substituiertes Aryl, oder
- eine Polyethylenglykol (PEG)-Kette oder eine Polyethylenglykolmonoalkyletherkette;
R3 und R4 unabhängig voneinander darstellen:
- Wasserstoff, oder
- eine Acylgruppe der Formel R5-CO-, worin R5 ein lineares oder verzweigtes, gesättigtes oder ungesättigtes, wahlweise mit einer oder mehreren wahlweise insbesondere mit Acyloxygruppen substituierten Arylgruppen substituiertes C1-C18-Alkyl darstellt, oder
- ein wahlweise, insbesondere mit Acyloxygruppen, substituiertes Aryl.

2. Zusammensetzung gemäß Anspruch 1, **dadurch gekennzeichnet, dass**:
R1 bezeichnet
- ein lineares oder verzweigtes, gesättigtes oder ungesättigtes, wahlweise mit einer oder mehreren Arylgruppen substituiertes C1-C18-Alkyl, oder
- ein wahlweise mit einer oder mehreren Hydroxy- und/oder Acyloxygruppen substituiertes Aryl;
R2 bezeichnet
- ein lineares oder verzweigtes, gesättigtes oder ungesättigtes C1-C18-Alkyl,
- ein wahlweise, insbesondere mit Acyloxygruppen, substituiertes Aryl,
- eine Polyethylenglykol (PEG)-Kette oder eine Polyethylenglykolmonoalkyletherkette.

3. Zusammensetzung gemäß einem der Ansprüche 1 oder 2, **dadurch gekennzeichnet, dass** R2 eine Polyethylenglykol (PEG)-Kette ist.

4. Zusammensetzung gemäß einem der Ansprüche 1 oder 2, **dadurch gekennzeichnet, dass** R1 -CH3 darstellt und R2 -CH3 oder -CH2-CH3 darstellt.

5. Zusammensetzung gemäß einem der Ansprüche 1 bis 4, **dadurch gekennzeichnet, dass** R3 und R4 unabhängig voneinander darstellen:
- eine Acylgruppe der Formel R5-CO-, worin R5 ein lineares oder verzweigtes, gesättigtes oder ungesättigtes, wahlweise mit einer oder mehreren wahlweise, insbesondere mit Acyloxygruppen, substituierten Arylgruppen substituiertes C1-C18-Alkyl darstellt, oder
- ein wahlweise, insbesondere mit Acyloxygruppen, substituiertes Aryl.

6. Zusammensetzung gemäß einem der Ansprüche 1 bis 5, **dadurch gekennzeichnet, dass** R3 und R4 beide eine Acetylgruppe CH3-CO- darstellen.

7. Zusammensetzung gemäß einem der vorhergehenden Ansprüche, **dadurch gekennzeichnet, dass** die Verbindung der Formel (I) einen Methylester des Triacetylleucodopachroms (II) oder einen Ethylester des Triacetylleucodopachroms (III) darstellt:

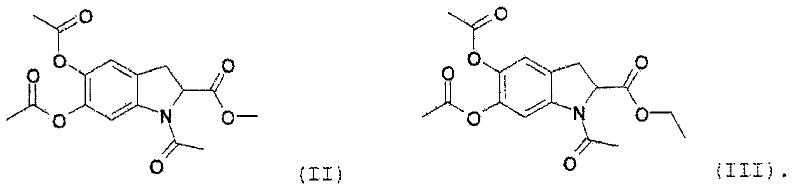

8. Zusammensetzung gemäß einem der vorhergehenden Ansprüche, **dadurch gekennzeichnet, dass** sie eines der Enantiomere oder ein Razemat (50/50) der zwei optisch aktiven Verbindungen der Formel (I) umfasst.

9. Zusammensetzung gemäß einem der vorhergehenden Ansprüche, **dadurch gekennzeichnet, dass** sie die Verbindung der Formel (I) in einer von 0,00001 bis 10 Gew.% und bevorzugt von 0,001 bis 5 Gew.% im Verhältnis zum Gesamtgewicht der Zusammensetzung reichenden Menge enthält.

10. Verfahren zum Reduzieren oder Verhindern von Pigmentierung und/oder Bleichen und/oder Aufhellen von Haut, die die Anwendung einer kosmetischen oder dermatologischen Zusammensetzung auf der Haut umfasst, **dadurch gekennzeichnet, dass** sie in einer physiologisch akzeptablen Umgebung mindestens eine Verbindung der Formel (I) enthält 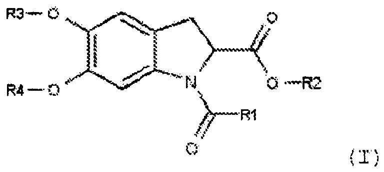 worin R1 darstellt:
- Wasserstoff, oder
- ein lineares oder verzweigtes, gesättigtes oder ungesättigtes, wahlweise mit einer oder mehreren Arylgruppen substituiertes C1-C18-Alkyl, oder
- ein wahlweise mit einer oder mehreren Hydroxy- und/oder Acyloxygruppen substituiertes Aryl;
R2 darstellt:
- Wasserstoff, oder
- ein lineares oder verzweigtes, gesättigtes oder ungesättigtes C1-C18-Alkyl, oder
- ein wahlweise, insbesondere mit Acyloxygruppen, substituiertes Aryl, oder
- eine Polyethylenglykol (PEG)-Kette oder eine Polyethylenglykolmonoalkyletherkette;
R3 und R4 unabhängig voneinander darstellen:
- Wasserstoff, oder
- eine Acylgruppe der Formel R5-CO-, worin R5 ein lineares oder verzweigtes, gesättigtes oder ungesättigtes, wahlweise mit einer oder mehreren wahlweise, insbesondere mit Acyloxygruppen, substituierten Arylgruppen substituiertes C1-C18-Alkyl darstellt, oder
- ein wahlweise, insbesondere mit Acyloxygruppen, substituiertes Aryl;
wobei die Zusammensetzung gemäß einem der Ansprüche 1 bis 9 ist.

11. Verwendung einer Verbindung der Formel (I) 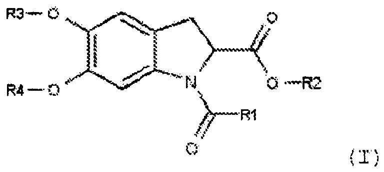 in der R1, R2, R3 und R4 wie zuvor in den Ansprüchen 1 bis 8 definiert sind,
als Depigmentierungsmittel und/oder Anti-Pigmentierungsmittel und/oder Bleichmittel und/oder Aufhellmittel.

12. Verwendung gemäß Anspruch 11 zur Herstellung einer zum Inhibieren der Melaminsynthese bestimmten Zusammensetzung.

13. Verbindung gemäß Formel (I') 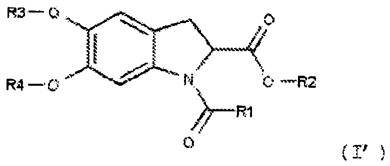 worin R1 darstellt:
- ein lineares oder verzweigtes, gesättigtes oder ungesättigtes, wahlweise mit einer oder mehreren Arylgruppen substituiertes C1-C18-Alkyl, oder
- ein wahlweise mit einer oder mehreren Hydroxy-, Acyloxygruppen substituiertes Aryl;
R2 darstellt:
- ein lineares oder verzweigtes, gesättigtes oder ungesättigtes C1-C18-Alkyl, oder
- ein wahlweise, insbesondere mit Acyloxygruppen, substituiertes Aryl, oder
- eine Polyethylenglykol (PEG)-Kette oder eine Polyethylenglykolmonoalkyletherkette;
R3 und R4 unabhängig voneinander darstellen:
- eine Acylgruppe der Formel R5-CO-, worin R5 ein lineares oder verzweigtes, gesättigtes oder ungesättigtes, wahlweise mit einer oder mehreren wahlweise, insbesondere mit Acyloxygruppen, substituierten Arylgruppen substituiertes C1-C18-Alkyl darstellt, oder
- ein wahlweise, insbesondere mit Acyloxygruppen, substituiertes Aryl.

14. Verbindung gemäß Anspruch 13, **dadurch gekennzeichnet, dass** R2 darstellt:
- eine lineare C3-C18-Alkylgruppe, oder
- eine Polyethylenglykol (PEG)-Kette oder eine Polyethylenglykolmonoalkyletherkette.

15. Verbindung gemäß einem der Ansprüche 13 oder 14, **dadurch gekennzeichnet, dass** die Verbindung der Formel (I') einen Propylester des Triacetylleucodopachroms der Formel (IV) darstellt: 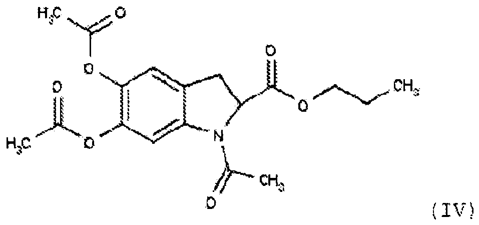 wahlweise in Form eines der Enantiomere oder eines Razemats (50/50) der zwei optisch aktiven Verbindungen der Formel (IV).

## Claims

1. A cosmetic or dermatological composition, **characterized in that** it contains, in a physiologically acceptable medium, at least one compound of formula (I) 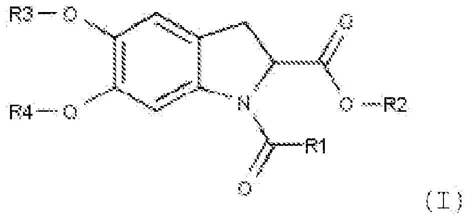 where R1 is
- a hydrogen, or
- a saturated or unsaturated, linear or branched C1-C18 alkyl, optionally substituted with one or more aryl groups, or
- an aryl optionally substituted with one or more hydroxyl and/or acyloxy groups;
R2 is:
- a hydrogen, or
- a saturated or unsaturated, linear or branched C1-C18 alkyl, or
- an aryl that is optionally substituted, in particular with acyloxy groups, or
- a polyethylene glycol (PEG) chain or a polyethylene glycol monoalkyl ether chain;
R3 and R4 are, independently of one another:
- a hydrogen, or
- an acyl group of formula R5-CO- where R5 is a saturated or unsaturated, linear or branched C1-C18 alkyl, optionally substituted with one or more aryl groups that are optionally substituted, in particular with acyloxy groups, or
- an aryl that is optionally substituted, in particular with acyloxy groups.

2. The composition as claimed in claim 1, **characterized in that**:
R1 denotes
- a saturated or unsaturated, linear or branched C1-C18 alkyl, optionally substituted with one or more aryl groups, or
- an aryl optionally substituted with one or more hydroxyl and/or acyloxy groups;
R2 denotes
- a saturated or unsaturated, linear or branched C1-C18 alkyl,
- an aryl that is optionally substituted, in particular with acyloxy groups,
- a polyethylene glycol (PEG) chain or a polyethylene glycol monoalkyl ether chain.

3. The composition as claimed in either one of claims 1 and 2, **characterized in that** R2 is a polyethylene glycol (PEG) chain.

4. The composition as claimed in either one of claims 1 and 2, **characterized in that** R1 is -CH₃ and R2 is -CH₃ or -CH₂-CH₃.

5. The composition as claimed in any one of claims 1 to 4, **characterized in that** R3 and R4 are, independently of one another:
- an acyl group of formula R5-CO- where R5 is a saturated or unsaturated, linear or branched C1-C18 alkyl, optionally substituted with one or more aryl groups that are optionally substituted, in particular with acyloxy groups, or
- an aryl that is optionally substituted, in particular with acyloxy groups.

6. The composition as claimed in any one of claims 1 to 5, **characterized in that** R3 and R4 are both CH₃-CO- acetyl groups.

7. The composition as claimed in any one of the preceding claims, **characterized in that** the compound of formula (I) is the methyl ester of triacetyl leucodopachrome (II) or the ethyl ester of triacetyl leucodopachrome (III):

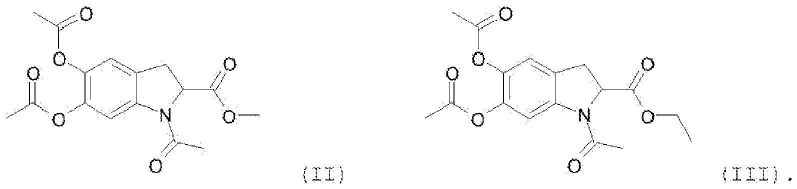

8. The composition as claimed in any one of the preceding claims, **characterized in that** it comprises one of the enantiomers or a racemic (50/50) mixture of two optically active compounds of formula (I).

9. The composition as claimed in any one of the preceding claims, **characterized in that** the compound of formula (I) is present in an amount ranging from 0.00001 to 10% by weight and preferably from 0.001 to 5% by weight relative to the total weight of the composition.

10. A process for reducing or preventing pigmentation and/or bleaching and/or lightening the skin, comprising the application to the skin of a cosmetic or dermatological composition, **characterized in that** it contains, in a physiologically acceptable medium, at least one compound of formula (I) 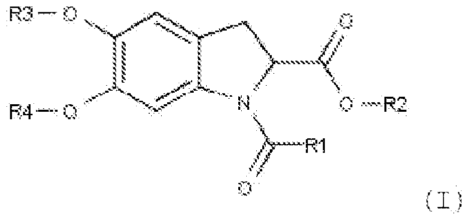 where R1 is
- a hydrogen, or
- a saturated or unsaturated, linear or branched C1-C18 alkyl, optionally substituted with one or more aryl groups, or
- an aryl optionally substituted with one or more hydroxyl and/or acyloxy groups;
R2 is:
- a hydrogen, or
- a saturated or unsaturated, linear or branched C1-C18 alkyl, or
- an aryl that is optionally substituted, in particular with acyloxy groups, or
- a polyethylene glycol (PEG) chain or a polyethylene glycol monoalkyl ether chain;
R3 and R4 are, independently of one another:
- a hydrogen, or
- an acyl group of formula R5-CO- where R5 is a saturated or unsaturated, linear or branched C1-C18 alkyl, optionally substituted with one or more aryl groups that are optionally substituted, in particular with acyloxy groups, or
- an aryl that is optionally substituted, in particular with acyloxy groups;
said composition being as claimed in any one of claims 1 to 9.

11. The use of a compound of formula (I) 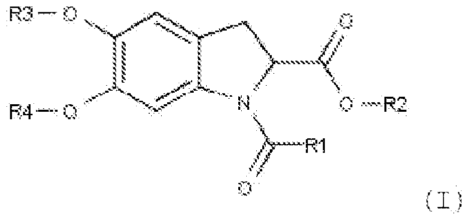 in which
R1, R2, R3 and R4 are as defined in claims 1 to 8, as a depigmenting and/or anti-pigmenting and/or bleaching and/or lightening agent.

12. The use as claimed in claim 11, for the manufacture of a composition intended to inhibit the synthesis of melanin.

13. A compound of formula (I') 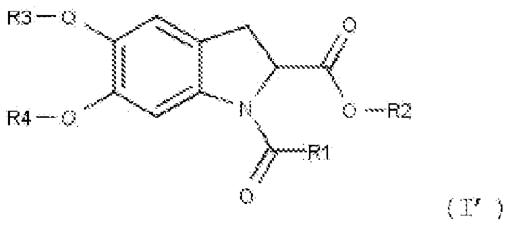 where R1 is
- a saturated or unsaturated, linear or branched C1-C18 alkyl, optionally substituted with one or more aryl groups, or
- an aryl optionally substituted with one or more hydroxyl or acyloxy groups;
R2 is:
- a saturated or unsaturated, linear or branched C3-C18 alkyl, or
- an aryl that is optionally substituted, in particular with acyloxy groups, or
- a polyethylene glycol (PEG) chain or a polyethylene glycol monoalkyl ether chain;
R3 and R4 are, independently of one another:
- an acyl group of formula R5-CO- where R5 is a saturated or unsaturated, linear or branched C1-C18 alkyl, optionally substituted with one or more aryl groups that are optionally substituted, in particular with acyloxy groups, or
- an aryl that is optionally substituted, in particular with acyloxy groups.

14. The compound as claimed in claim 13, **characterized in that** R2 is:
- a linear C3-C18 alkyl group, or
- a polyethylene glycol (PEG) chain or a polyethylene glycol monoalkyl ether chain.

15. The compound as claimed in either one of claims 13 and 14, **characterized in that** the compound of formula (I') is the propyl ester of triacetyl leucodopachrome of formula (IV) 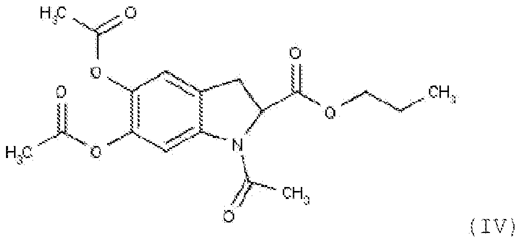 optionally in the form of one of the enantiomers or a racemic (50/50) mixture of two optically active compounds of formula (IV).
